# EUROPEAN PATENT APPLICATION

(11) **EP 3 011 922 A1**
(43) Date of publication of application: **27.04.2016**
(21) Application number: 13887142.1
(22) Date of filing: 18.06.2013
(51) Int. Cl.: A61B 18/00, A61B 18/12, A61N 7/00

(54) **POWER SUPPLY DEVICE FOR SURGICAL INSTRUMENT, USING ULTRASONIC WAVES**

(71) Applicant: Kim, Eungkook, Seoul 137-831 (KR)
(72) Inventor: Kim, Eungkook, Seoul 137-831 (KR)
(74) Representative: Lawrence, John
(86) International application number: PCT/KR2013/005371
(87) International publication number: WO 2014/204026

(57) **Abstract**

The present invention relates to a device for supplying power to a surgical instrument for amputating a surgical site or stopping bleeding by using ultrasonic waves, comprising a current control unit for controlling the intensity of current flowing into a vibrator, which is provided at the surgical instrument to control the intensity of ultrasonic waves oscillated at the surgical instrument, wherein the current control unit controls the intensity of current flowing into the vibrator such that weakening the intensity of current, flowing into the vibrator, for a predetermined time period can be periodically repeated.

## Description

### TECHNICAL FIELD

The present invention relates to a device for supplying power to a surgical apparatus using ultrasonic waves, more particularly, to a device for supplying power to a surgical operation apparatus using heat generation of ultrasonic wave. The degree of amputating a surgical site or stopping bleeding can be freely and easily adjusted according to the intension of a user, while minimizing the heat generation of the surgical apparatus using ultrasonic waves, so that the user can operate the apparatus with improved convenience.

### BACKGROUND ART

Recently, surgical apparatuses using ultrasonic waves are being developed. Among them, the surgical apparatus such as Harmonic Scalpel which can cut and stop bleeding at the same time is playing an important role in laparoscopic surgery.

However, those apparatuses equipped with power supply devices which applies power at a constant intensity of current are commercialized, and there is yet no power supply device which can supply power suitable for a surgical apparatus using ultrasonic waves which have been developed.

Accordingly, since the surgical apparatuses using ultrasonic waves use power supply devices which can supply a constant current only, such surgical apparatuses are liable to easy failure due to excessive heat generation.

Such problem also involves the problem that the cutting and/or stopping bleeding at a surgical site cannot be freely controlled by the intention of the user.

In addition, newly developed surgical apparatuses using ultrasonic waves provide for a switch on the surgical apparatus for controlling current even though the apparatus is designed to provide a constant current.

However, such configuration also cannot dissolve the problem that cannot minimize heat generation. In addition, since a user must control the current using the control switch continuously, leading to fatigue in the hand, deteriorating user convenience, the technical problem still exists to be dissolved.

### DETAILED DESCRIPTION OF THE INVENTION

### TECHNICAL OBJECTS

To solve above described problem, an aspect of the present invention is to provide a power supply device for a surgical apparatus using ultrasonic waves which can control the speed of degree of cutting and stopping bleeding at a surgical site according to the intention of a user, while enables to minimize heat generation of the surgical apparatus using ultrasonic waves.

In addition, another aspect of the present invention is to provide a power supply device for a surgical apparatus using ultrasonic wave which can improve user convenience.

The invention is not restricted to the embodiments set forth herein. The above and other aspects of the invention will become apparent to those skilled in the art to which the invention pertains by referencing the detailed description of the invention below.

### MEANS FOR ACHIVING THE TECHNICAL OBJECT

The technical object of the present invention to solve above described problem is to provide a power supply device for a surgical apparatus which can cut and stop bleeding of a surgical site using ultrasonic waves, the device comprising: a current regulating unit which controls the intensity of the electric current flowing into the ultrasonic vibrator of the surgical apparatus in order to control the intensity of the ultrasonic waves, wherein the current regulating unit can control the intensity of the current by periodically reducing the current flowing into the vibrator.

Here, the current regulating unit may be activated after the vibrator has started vibration so that the initial vibration of the vibrator may not be disturbed by the control of the current flowing into the vibrator by the current regulating unit.

Meanwhile, regarding the intensity of the current flowing into the vibrator, a minimum intensity of the current which enables the vibrator to keep vibrating is defined as a first current intensity, a maximum intensity of the current which enables the vibrator to keep vibrating is defined as a third current intensity, and a certain intensity of the current determined by a user, between the first and the third intensities, is defined as a second current intensity, where, if the user intends to cut a surgical site using the surgical apparatus, the current regulating unit controls the intensity of the current flowing into the vibrator between the second and the third intensities, with the second current intensity repeats periodically for a predetermined time period, and if the user intends to stop bleeding from the surgical site, the current regulating unit controls the intensity of the current flowing into the vibrator between the second and the first intensities, with the first current intensity repeats periodically for a predetermined time period.

Here, the intensity of the current flowing into the vibrator can be defined in a plurality of intensity levels according to the intension of the user between the first and the third current intensities, and can configure that a current intensity lower than that of the second intensity repeats periodically for a predetermined time period.

Meanwhile, the current regulating unit, if the user intends to cut a surgical site using the surgical apparatus using ultrasonic wave, first apply a current whose intensity is higher than the intensity required to initiate vibration of the vibrator into the vibrator to start the vibration, and reduce the intensity of the current periodically for a predetermined time period.

Here, let the intensity of the current required to initiate the vibration of the vibrator is between from about 0.55 A to about 0.65 A, then the intensity of the current higher than the intensity of the current required to initiate the vibration of the vibrator, for example, is between from about 1.30 A to about 1.40 A, and the intensity of the current flowing into the vibrator which is reduced for a predetermined time can be between from about 0.30 A to about 0.40 A.

In addition, if the user intends to stop bleeding of the surgical site by reducing the intensity of the ultrasonic waves generated by the surgical apparatus, the current regulating unit can control the current to a level lower than that required to start vibration of the vibrator flowing into the vibrator periodically for a predetermined time period.

Here, let the intensity of the current required to initiate the vibration of the vibrator is between from about 0.55 A to about 0.65 A, then the intensity of the current flowing into the vibrator which is lower than the level required to initiate vibration of the vibrator can be between from about 0.30 A to about 0.40 A.

Meanwhile, the operation of the current regulating unit can be controlled by an independent controller isolated from the surgical apparatus, in addition, the controller can be configured to control the current regulating unit by a radio control.

### EFFECTS OF THE INVENTION

According to the power supply device in accordance with an embodiment of the present invention for a surgical apparatus using ultrasonic waves, the speed and degree of cutting and stopping bleeding of a surgical site can be controlled freely and easily by the user by controlling the intensity of the current flowing into the vibrator via the current regulating unit, and can minimize heat generation of the surgical apparatus by preventing unreasonably high vibration and ultrasonic wave generation.

Since the current regulating unit can be so configured as to be able to be controlled by an independent controller isolated from the surgical apparatus, in addition, the user of the surgical apparatus can be free from the operation of the controller, improving user convenience.

### BRIEF DESCRIPTION OF THE DRAWINGS

FIG. 1 is an embodiment of the power supply device in accordance with an embodiment of the present invention and a surgical apparatus using ultrasonic waves which receives power from the power supply device.
FIG. 2 is a view of a modification of the controller included in the embodiment of FIG. 1 and a front, cut of view of the surgical apparatus using ultrasonic waves.
FIGs. 3 to 7 are the graphs for describing the operation of the current regulating unit included in an embodiment of the power supply device for a surgical apparatus using ultrasonic waves.

### <Numbering Scheme of the Major Parts of the Drawings>

100 : surgical apparatus using ultrasonic waves
110 : vibrator
112 : cutter
120 : ultrasonic wave generating switch
130 : jaw switch
132: jaw
200 : embodiment of the present invention including a current regulating unit
210 : current regulating unit
220 : current control switch
230, 230' : controller;
240 : display

### MODE FOR CARRYING OUT THE INVENTION

An embodiment of the single port surgical trocar in accordance with the present invention is described herein below by referring to the accompanying drawings. In the description of the present invention, known functions and/or configuration can be omitted in order to clarify the spirit of the present invention.

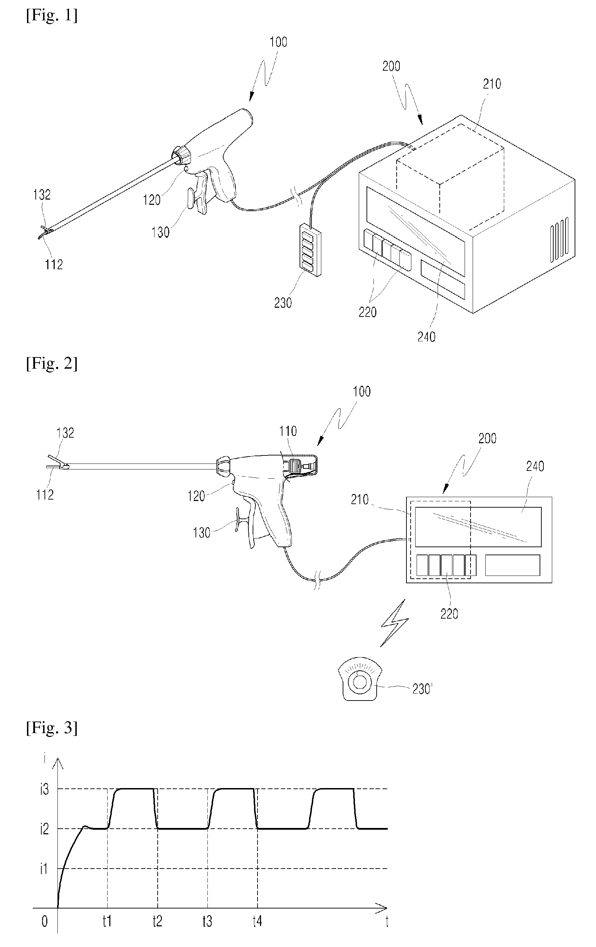

To begin with, a surgical apparatus using ultrasonic waves is described before describing an embodiment of the power supply device for a surgical apparatus using ultrasonic waves in accordance with an embodiment of the present invention by referring to FIGs. 1 and 2.

Here, FIG. 1 is an embodiment of the power supply device in accordance with an embodiment of the present invention and a surgical apparatus using ultrasonic waves which receives power from the power supply device and FIG. 2 is a view of a modification of the controller included in the embodiment of FIG. 1 and a front, cut of view of the surgical apparatus using ultrasonic waves.

As shown in FIG. 1 and 2, a surgical apparatus using ultrasonic waves 100 cuts or stops bleeding at a surgical site by delivering ultrasonic waves from a cutting member 112.

Here, the basic configuration to deliver ultrasonic waves to the cutting member 112 includes a vibrator 110 which is configured with electrodes arranged in between a plurality of ceramics to allow current flow which controls the degree of the vibration.

As an on-off switch for the vibrator 110, an ultrasonic wave generation switch 120 is provided. As described earlier in the Background section, a plurality of ultrasonic wave generation switches 120 are provided in the conventional art, in order to control the degree of the vibration of the vibrator 110, differently, an embodiment 200 of the power supply device for a surgical apparatus in accordance with the present invention may be provided with only one.

In addition, the surgical apparatus using ultrasonic waves 100 can be provided with a jaw 132 adjacent to the cutting member 112 to so that the cutting member 112 can easily cut and stop bleeding at a surgical site, wherein the jaw 132 can be controlled by a jaw switch 130 provided adjacent to the ultrasonic wave generation switch 120.

The surgical apparatus using ultrasonic waves 100 so configured, as described in the Background section, is subject to heat generation in the course of generating and transmitting ultrasonic waves, In addition, the user has to keep holding the device for a long time, feeling fatigue, raising a demand for an improvement for user convenience.

Accordingly, the significance of the embodiment 200 of a power supply device for a surgical apparatus using ultrasonic waves in accordance with the present invention is that it can dissolve above described problems.

Referring to FIGs. 1 and 2, an embodiment 200 of the power supply device for a surgical apparatus using ultrasonic waves in accordance with the present invention comprises a current regulating unit 210.

Here, the current regulating unit 210 is configured to control the intensity of the current flowing into the vibrator 110 to regulate the intensity of the ultrasonic waves generated at the surgical apparatus using ultrasonic waves 100.

The function of the current regulating unit 210 is to regulate the intensity of the current flowing into the vibrator 110 in such a manner that it can reduce the intensity of the current flowing into the vibrator 110 periodically for a predetermined time period. Further details will be described referring to FIGs. 3 to 7, after describing an additional configuration to the embodiment 200 of the power supply device for a surgical apparatus using ultrasonic waves in accordance with the present invention.

In addition, an embodiment 200 of the power supply device for a surgical apparatus using ultrasonic waves in accordance with the present invention can further comprise a current control switch 220, a controllers 230 and 230', and a display 240 in addition to the current regulating unit 210.

Here, the current control switch 220 is configured to be connected to the current regulating unit 210 and able to control the current regulating unit 210 in an embodiment 200 in accordance of the power supply device in accordance with the present invention for a surgical apparatus using ultrasonic waves configured in a box type.

Meanwhile, the controllers 230 and 230' are provided to control the operation of the current regulating unit 210 even when the user or an assistant who operates the current control switch 220 is not within the arm's reach to the embodiment 200 of the power supply device for a surgical apparatus using ultrasonic waves in accordance with the present invention.

These controllers 230 and 230' shall be separated from the surgical apparatus using ultrasonic waves 100 so that it is not controlled by the surgical apparatus using ultrasonic waves 100 held by the user to further improve user convenience.

In addition, as shown in FIG. 1, the controllers 230 and 230' can be a wired controller 230 with buttons, or as shown in FIG. 2, a radio controller 230' with a dial. It would be apparent for those skilled in the art that the configuration of these controllers 230 and 230' can be diverse, and thus, they do not limit the scope of the present invention.

Meanwhile, the display 240 is provided to show the condition of the embodiment 200 of the power supply device for a surgical apparatus using ultrasonic waves in a visible manner, including the information of the control of the current regulating unit 210 as well as other information related with the surgical apparatus using ultrasonic waves 100 in a diversified way. it would be also apparent that the configuration of the display can be modified in various ways without limiting the scope of the present invention.

Herein below, of the embodiment 200 of the power supply device for a surgical apparatus using ultrasonic waves in accordance with the present invention, the current regulating unit 210 which was not described in detail is described by referring to FIGs. 3 to 7. In addition, the functionality and effects of the embodiment 200 of the power supply device for a surgical apparatus using ultrasonic waves in accordance with the present invention are described in further details.

Here, FIGs. 3 to 7 are the graphs for describing the operation of the current regulating unit included in an embodiment of the power supply device for a surgical apparatus using ultrasonic waves.

As shown in FIGs. 3 to 7, the current regulating unit 210 regulates the intensity of the current to i2 which flows into the vibrator 110 so that the vibrator 110 can start vibration.

This is intended to control the intensity of the current flowing into the vibrator 110 by the current regulating unit 210 to allow the vibrator to start generating ultrasonic waves and the current regulating unit 210 begins operation after the vibrator 110 has started vibration.

After the vibrator 110 has received a current whose intensity is sufficient to start vibration of the vibrator 110, the current regulating unit 210 regulates the intensity of the current flowing into the vibrator 110, as shown in FIGs. 3 to 7, in a diversity of manners. A general description is provided before describing the cases of FIGs. 3 to 7 respectively.

Beforehand, the intensities of the current depicted in FIGs. 3 to 7 are defined: A first current intensity i1 is the minimum current intensity, of the current flowing into the vibrator 110, which enables continuous generation of ultrasonic waves, and the third current intensity i3 is the maximum current intensity, of the current flowing into the vibrator 110, which enables continuous generation of ultrasonic waves. In addition, a second current intensity i2 is an intensity defined by the user between the first current intensity i1 and the third current intensity i3 according to the user's reference. In FIGs. 3 to 7, it is assumed that the user has defined the second current intensity i2 as the intensity of current with which the vibrator 110 can start vibration.

Describing the current regulating unit 210 assuming the intensities of the currents so defined and depicted in FIGs. 3 to 7, the current regulating unit 210 can be described largely in two cases.

Firstly, if the user intends to cut a surgical site using the surgical apparatus using ultrasonic waves 100, the intensity of the ultrasonic waves must be high to burn away the site, the intensity of the current flowing into the vibrator 110 shall be between the second current intensity i2 and the third current intensity i3, and the second current intensity i2 shall repeated periodically for a predetermined time.

Next, if the user intends to stop bleeding at the surgical site using the surgical apparatus using ultrasonic waves 100, the intensity of the ultrasonic waves must not be high to coagulate the blood, the intensity of the current flowing into the vibrator 110 shall be between the first current intensity i1 and the second current intensity i2 and the first current intensity i1 shall be repeated periodically for a predetermined time.

Here, the predetermined time is defined by the manufacturer or the user taking into consideration the material, shape, and size of the vibrator 110, and the intensity of the current and time of the current flowing into the vibrator 110, during which the intensity of the current flowing into the vibrator 110 is controlled at a low level in order to protect the vibrator 110 from any problem during vibration.

For example, if the vibrator 110 is a ceramic disc having a thickness of about 2 mm, and about 0.78 A of current is applied to the vibrator 110 for about 2 s, the predetermined time for applying the current to the vibrator 110 can be set up by about 1 s, and during this time, the intensity of the current flowing into the vibrator 110 can be reduced to about 0.67 A.

This adjustment can be compared to an audio speaker system connected to an amplifier. If the speaker system keeps outputting at the maximum level, the vibrating disc can be damaged. To prevent this, the output from the amplified is reduced periodically. (It would be apparent for those skilled in the art that this example of a speaker system is solely for a metaphor, which is different from the principle of the present invention. Those skilled in the art shall read the detailed description of the present invention for full understanding.)

In addition, the intensities of the current flowing into the vibrator 110 need not be classified into three levels, from i1 to i3. That is, the intensity of the current flowing into the vibrator 110 can be defined in a plurality of intensity levels according to the intension of the manufacturer or user between the first current intensity i1 and the third current intensity i3, and the current regulating unit 210 will control the current input at a lower level than the second current intensity i2 repeating periodically for a predetermined time period.

In addition, according to the above description, it may be misunderstood that, the cutting and stopping bleeding at a surgical site using the surgical apparatus using ultrasonic waves 100 are divided independently, since the energy levels required for cutting and stanching are the same, the description is focused on the surgical site only, and as the case may be, stanching may be applied during cutting and vice versa, which will be apparent for those skilled in the art.

The description above is described in further details herein below referring to the accompanying drawings.

If the user intends to cut a surgical site using the surgical apparatus using ultrasonic waves 100 by increasing the intensity of the ultrasonic waves, as shown in FIG. 3, the user will apply a current of a third intensity i3 to the vibrator 110, which is higher that the intensity of the current required to start vibration of the vibrator 110 (exemplified as a second intensity of current i2), and the intensity of the current applied to the vibrator 110 can be reduced repeatedly for a predetermined time period, by the control function of the current regulating unit 210.

Here, to exemplify the intensities of the currents from i1 to i3 with a concrete figures, assuming the intensity of the current required to initiate the vibration of the vibrator 110 is between from about 0.55 A to about 0.65 A, then the intensity of the current higher than the intensity of the current required to initiate the vibration of the vibrator 110 is between from about 1.30 A to about 1.40 A, and the intensity of the current flowing into the vibrator 110 reduced for a predetermined time can be between from about 0.30 A to about 0.40 A.

These values are exemplified by taking into consideration the frequency of the ultrasonic waves delivered at the cutting member 112 of the surgical apparatus using ultrasonic waves 100, and the performance and function of the circuits included in the exemplary embodiment 200 of the power supply device for a surgical apparatus using ultrasonic waves in accordance with the present invention. Those skilled in the art will be able to modify and implement these values according to these conditions without significant difficulties, and it would be apparent that these values, in addition to the values provided below, shall not limit the scope of the present invention.

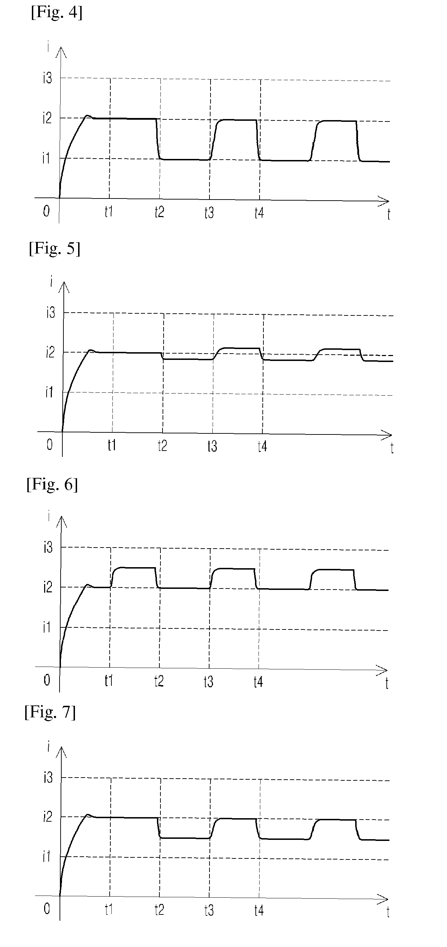

If the user intends to stanch a surgical site using the surgical apparatus using ultrasonic waves 100 by decreasing the intensity of the ultrasonic waves, as shown in FIG. 4, the user will be able to control the current regulating unit 210 so that a current of a first intensity i1 is applied to the vibrator 110, which is lower that the intensity of the current required to start vibration of the vibrator 110 (exemplified as a second intensity of current i2), and the first intensity of current i1 can be applied to the vibrator 110 repeatedly for a predetermined time period, by the control function of the current regulating unit 210.

Here, to exemplify the intensities of the currents from i1 to i3 with a concrete figures, assuming the intensity of the current required to initiate the vibration of the vibrator 110 is between from about 0.55 A to about 0.65 A, then the intensity of the current lower than the intensity of the current required to initiate the vibration of the vibrator 110 is between from about 0.30 A to about 0.40 A.

In addition, even if the purpose of the surgical apparatus using ultrasonic waves 100, that is, cutting or stopping bleeding of a surgical site, is not clear, the current regulating unit 210 can be controlled to control the current flowing into the vibrator 110 as shown in FIG. 5 to 7.

That is, as shown in FIG. 5, the current regulating unit 210 can be operated so that the current oscillates from high to low levels of intensity with reference to the intensity of the current required to start vibration of the vibrator 110 (exemplified by the second current intensity i2). This type of operation will be useful when the surgical apparatus using ultrasonic waves 100 is used to stop bleeding from a portion of the surgical site cut with the apparatus.

In addition, as shown in FIG. 6, the intensity of the current flowing into the vibrator 110 is controlled in a similar manner with the case illustrated in FIG. 3, having the intensity of the third current i3 be lower than that depicted in FIG. 3 and applied to the vibrator 110, and then reducing the intensity of the current applied to the vibrator 110 periodically, using the current regulating unit 210. The purpose of use of the surgical apparatus using ultrasonic waves in this case will be in between the purpose of the surgical apparatus using ultrasonic waves 100 described by referring to FIG. 3 and the purpose of the surgical apparatus using ultrasonic waves 100 described by referring to FIG. 5.

In addition, as shown in FIG. 7, the intensity of the current flowing into the vibrator 110 is controlled in a similar manner with the case illustrated in FIG. 4, having the intensity of the first current i1 be higher than that depicted in FIG. 4 and applied to the vibrator 110 repeatedly in a periodic manner, using the current regulating unit 210. The purpose of use of the surgical apparatus using ultrasonic waves in this case will be in between the purpose of the surgical apparatus using ultrasonic waves 100 described by referring to FIG. 4 and the purpose of the surgical apparatus using ultrasonic waves 100 described by referring to FIG. 5.

In accordance with an embodiment 200 of the present invention described above, using an power supply device for a surgical apparatus using ultrasonic waves the speed and degree of cutting and stopping bleeding of a surgical site can be controlled by the intensity of the current flowing into the vibrator 110 via a current regulating unit 210, according to the intension of the user freely and easily. In addition, the heat generation in the surgical apparatus using ultrasonic waves 100 can be minimized by preventing excessive vibration and ultrasonic wave generation at the cutting member 112, which can improve the durability of the surgical apparatus using ultrasonic waves 100.

In addition, since the current regulating unit 210 can be controlled with the controllers 230 and 230' separate from the embodiment 200 of the power supply device for a surgical apparatus using ultrasonic waves 100 in accordance with the present invention, the user need not control the generation and intensity of the ultrasonic waves generated in the surgical apparatus using ultrasonic waves, improving user convenience.

In addition, even when the surgical apparatus using ultrasonic waves 100 and the embodiment 200 of the power supply device for a surgical apparatus using ultrasonic waves in accordance with the present invention are setup on a robot system, the speed and degree of cutting and stopping bleeding at a surgical site can be precisely controlled according to the intension of the user freely and easily. In addition, the durability of the surgical apparatus using ultrasonic waves 100 and the robot system mounting the same can be improved by preventing excessive vibration and ultrasonic wave generation at the vibrator 110 or cutting member 112.

In addition, when the surgical apparatus using ultrasonic waves 100 and the embodiment 200 of the power supply device for a surgical apparatus using ultrasonic waves in accordance with the present invention is mounted on a robot system, the current regulating unit 210 can still be controlled by separate controllers 230 and 230', still improving the user convenience.

From the foregoing it is believed that those skilled in the pertinent art will recognize the meritorious advancement of this invention and will readily understand that while the present invention has been described in association with a preferred embodiment thereof, and in the accompanying drawings, numerous changes and modifications may be made therein by those skilled in the art without departing from the spirit and scope of this invention. Therefore, the modifications and changes shall not be interpreted independently from the technical spirit and point of view, and the modified embodiments shall be interpreted to be within the scope of the present invention.

## Claims

1. A power supply device for a surgical apparatus for cutting or stopping bleeding at a surgical site using ultrasonic waves, comprising:
a current regulating unit for controlling the intensity of the electric current flowing in to a vibrator provided in the surgical apparatus in order to control the intensity of the ultrasonic waves generated by the surgical apparatus,
wherein the current regulating unit, is **characterized by** controlling the intensity of the current flowing into the vibrator so that the intensity of the current flowing into the vibrator is reduced periodically for a predetermined time.

2. The power supply device for a surgical apparatus using ultrasonic waves of claim 1,
wherein the current regulating unit, is **characterized by** being activated after the vibrator has started vibration in order to prevent the initiation of the vibration by controlling the intensity of the current flowing into the vibrator.

3. The power supply device for a surgical apparatus using ultrasonic waves of claim 2, of the intensity levels of the current flowing into the vibrator, let the first current intensity is a minimum intensity enabling continuous generation of ultrasonic waves, and let the third current intensity is a maximum intensity enabling continuous generation of ultrasonic waves, and a second current intensity is between the first and third intensities, defined by the user, wherein the current regulating unit, if the user intends to cut a surgical site using the surgical apparatus, shall control the intensity of the current flowing into the vibrator remain between the second and the third current intensities, and the intensity of the second current intensity varies periodically for a predetermined time period, if the user intends to stanch a surgical site using the surgical apparatus, shall control the intensity of the current flowing into the vibrator remain between the first and second current intensities, and the intensity of the first current intensity varies periodically for a predetermined time period.

4. The power supply device for a surgical apparatus using ultrasonic waves of claim 3,
wherein the intensity of the current flowing into the vibrator is divided into a plurality of intensity levels according to the determination of the user between the first current intensity and the third current intensity, **characterized in that**, a current having an intensity lower than the second current intensity appears periodically for a predetermined time period.

5. The power supply device for a surgical apparatus using ultrasonic waves of claim 2,
wherein the current regulating unit, if the user intends to cut a surgical site using the surgical apparatus using ultrasonic wave, first apply a current whose intensity is higher than the intensity required to initiate vibration of the vibrator into the vibrator to start the vibration, and reduce the intensity of the current periodically for a predetermined time period.

6. The power supply device for a surgical apparatus using ultrasonic waves of claim 5,
wherein, letting the intensity of the current required to initiate the vibration of the vibrator is between from about 0.55 A to about 0.65 A, then the intensity of the current higher than the intensity of the current required to initiate the vibration of the vibrator, for example, is between from about 1.30 A to about 1.40 A, and the intensity of the current flowing into the vibrator which is reduced for a predetermined time can be between from about 0.30 A to about 0.40 A.

7. The power supply device for a surgical apparatus using ultrasonic waves of claim 2, if the user intends to stop bleeding of the surgical site by reducing the intensity of the ultrasonic waves generated by the surgical apparatus, the current regulating unit can control the current to a level lower than that required to start vibration of the vibrator flowing into the vibrator periodically for a predetermined time period.

8. The power supply device for a surgical apparatus using ultrasonic waves of claim 7,
wherein, let the intensity of the current required to initiate the vibration of the vibrator is between from about 0.55 A to about 0.65 A, then the intensity of the current flowing into the vibrator which is lower than the level required to initiate vibration of the vibrator can be between from about 0.30 A to about 0.40 A.

9. The power supply device for a surgical apparatus using ultrasonic waves of any one of claim 1 to claim 8, wherein the current regulating unit, is **characterized by** being controlled by a separate controller from the surgical apparatus.

10. The power supply device for a surgical apparatus using ultrasonic waves of 9, wherein the controller is **characterized by** being configured to control the operation of the current regulating unit via a radio communication.
